# FASCICULE DE BREVET EUROPEEN

(11) **EP 1 267 922 B1**
(45) Date de publication et mention de la délivrance du brevet: **15.06.2005**
(21) Numéro de dépôt: 01917183.4
(22) Date de dépôt: 19.03.2001
(51) Int. Cl.: A61K 39/385, A61K 39/116

(54) **CONJUGUES POLYSACCHARIDIQUES DU PNEUMOCOQUE A USAGE VACCINAL CONTRE LE TETANOS ET LA DIPHTERIE**
POLYSACCHARIDKONJUGATE VON PNEUMOKOKKEN ALS IMPFSTOFF GEGEN TETANUS UND DIPHTERIE
PNEUMOCOCCUS POLYSACCHARIDE CONJUGATES FOR USE AS VACCINE AGAINST TETANUS AND DIPHTHERIA

(30) Priorité: 17.03.2000 FR 0003407
(43) Date de publication de la demande: 02.01.2003
(73) Titulaire: Sanofi Pasteur, 69367 Lyon Cedex 07 (FR)
(72) Inventeur: SCHULZ, Dominique, F-69004 Lyon (FR)
(74) Mandataire: Ayroles, Marie-Pauline
(86) Numéro de dépôt international: PCT/FR2001/000820
(87) Numéro de publication internationale: WO 2001/068128

(56) Documents cités:
- WO-A-96/40242
- WO-A-98/51339
- FR-A- 2 682 388
- US-A- 4 619 828
- SCHNEERSON R ET AL: "QUANTITATIVE AND QUALITATIVE ANALYSIS OF SERUM ANTIBODIES ELICITED IN ADULTS BY HAEMOPHILUS INFLUENZAE TYPE B AND PNEUMOCOCCUS TYPE 6A CAPSULAR POLYSACCHARIDE-TETANUS TOXOID CONJUGATES" INFECTION AND IMMUNITY,US,AMERICAN SOCIETY FOR MICROBIOLOGY. WASHINGTON, vol. 52, no. 2, 1 mai 1986 (1986-05-01), pages 519-528, XP000651787 ISSN: 0019-9567 cité dans la demande
- SHAMS HOMAYOUN ET AL: "The effect of conjugation on immunogenicity and potency of protein carriers of polyribosylribitol phosphate (PRP) conjugated vaccines in the mouse model." APMIS, vol. 106, no. 5, mai 1998 (1998-05), pages 526-534, XP000971158 ISSN: 0903-4641 cité dans la demande

## Description

La présente invention se rapporte à l'utilisation de combinaisons vaccinales pour la prévention du tétanos et/ou de la diphtérie.

### Etat de la technique

Pour réduire le nombre d'injections on associe de plus en plus les antigènes vaccinaux entre eux sous la forme de compositions vaccinales multivalentes. Bien que cette approche soit avantageuse on a rapporté des interactions négatives entre les antigènes avec pour conséquence une baisse relative de l'immunogénicité d'un ou plusieurs composants. Ce risque est d'autant plus important que le nombre d'antigènes encore appelés "valences" est important. On connaît notamment des vaccins multivalents comprenant les valences de la diphtérie et du tétanos. La combinaison des antigènes de la diphtérie, du tétanos et de la coqueluche avec ceux du virus de la polio entraîne une diminution de la réponse immune à la coqueluche.

On cherche à améliorer le vaccin contre le pneumocoque. Un des candidats est le vaccin multivalent tel que décrit dans WO 98/51339. Il comprend des polysaccharides capsulaires provenant de différents sérotypes de *Streptococcus pneumoniae* conjugués à l'anatoxine tétanique et/ou à l'anatoxine diphtérique.

On sait qu'en conjuguant des polysaccharides à un porteur protéique on favorise le développement d'une immunité T dépendante dirigée contre les polysaccharides. On sait également que l'immunogénicité du(des) porteur(s) est généralement diminuée.

Homayoun S. et al., APMIS (1998); 106; 526-534 a rapporté que la réponse anticorps spécifique vis à vis de l'anatoxine tétanique variait considérablement selon que cette protéine était couplée à un polysaccharide capsulaire d'*Haemophilus influenzae type b* de bas poids moléculaire ou de haut poids moléculaire.

Anderson P et al., J. Immunol. (1989), 142, 2464-2468 a montré également que la taille du polysaccharide pouvait influer sur la réponse à l'anatoxine diphtérique lorsque celle ci est utilisée comme molécule porteuse.

Schneerson R. et al., (Infection and Immunity (1986); 52, 519-528) a montré qu'il faut administrer une quantité importante d'anatoxine tétanique conjuguée au polysaccharide capsulaire du pneumocoque de sérotype 6B (≥ 80µg) pour observer une réponse anticorps dirigée contre la protéine porteuse. Cette dose de protéine est comparativement beaucoup plus importante que la dose couramment utilisée pour vacciner contre le tétanos, qui est comprise entre 15µg et 30 µg ou encore entre 5 et 10 Lf d'anatoxine tétanique.

Par conséquent, il est généralement admis que si la conjugaison d'un polysaccharide à un porteur protéique est bénéfique pour le développement d'une immunité dirigée contre le polysaccharide, elle gêne par contre le développement d'une réponse immune dirigée contre le porteur. Un vaccin contenant un conjugué polysaccharide couplé à l'anatoxine tétanique ou diphtérique, induit une immunité protectrice contre l'organisme pathogène exprimant à sa surface ledit polysaccharide, mais n'induit pas d'immunité complète vis à vis du tétanos ou de la ) diphtérie. Il faut un apport additionnel d'anatoxine libre au vaccin conjugué pour l'obtenir. A titre d'exemple, on peut citer le vaccin conjugué contre *haempohilus influenzae* dans lequel le polysaccharide est conjugué à l'anatoxine tétanique. Pour obtenir une protection contre le tétanos, il faut associer ce vaccin à un vaccin anti tétanique classique. Ainsi, un vaccin polysaccharidique conjugué à l'anatoxine tétanique ou l'anatoxine diphtérique ne représente donc pas une bonne solution pour obtenir une immunité complète vis à vis du porteur protéique puisqu'il faut l'associer au vaccin anti tétanique ou anti diphtérique.
De façon surprenante, on a maintenant identifié un vaccin polysaccharidique conjugué à un porteur protéique permettant d'obtenir une immunité protectrice vis à vis dudit porteur.

### Résumé de l'invention

A cet effet, la présente invention concerne l'utilisation d'une composition comprenant «n» conjugués polysaccharidiques constitués individuellement d'un polysaccharide capsulaire de *Streptococcus pneumoniae* couplé à l'anatoxine tétanique, pour la fabrication d'un vaccin protégeant contre les infections à *Clostridium tetani*, composition dans laquelle:
*(1)* les « n » polysaccharides capsulaires des « n » conjugués polysaccharidiques sont tous différents,
*(2)* « n » est supérieur ou égal à 2 et inférieur ou égal à 23,
*(3)* la quantité totale d'anatoxine tétanique conjuguée présente dans une dose vaccinale, est de 6 à 40 µg ; et
*(4)* la quantité totale d'anatoxine tétanique conjuguée présente dans une dose vaccinale est suffisante pour induire une protection contre les infections à *Clostridium tetani*.

Dans un mode de réalisation, la quantité totale d'anatoxine tétanique conjuguée présente dans une dose vaccinale est de 10 à 25 µg.

Dans un autre mode de réalisation, « n » est entre 4 et 11.

Dans un mode de réalisation particulier, « n » est égal à 11 et les « n » polysaccharides capsulaires des « n » conjugués polysaccharidiques sont les polysaccharides des sérotypes 1, 3, 4, 5, 6B, 7F, 9V, 14, 18C, 19F et 23F.

L'invention concerne aussi l'utilisation d'une composition comprenant « p » conjugués polysaccharidiques constitués individuellement d'un polysaccharide capsulaire de *Streptococcus pneumoniae* couplé à l'anatoxine diphtérique, pour la fabrication d'un vaccin protégeant contre les infections à *Corynebactérium diphteriae*, composition dans laquelle:
*(1)* les « p » polysaccharides capsulaires des « p » conjugués polysaccharidiques sont tous différents,
*(2)* « p » est supérieur ou égal à 2 et inférieur ou égal à 15,
*(3)* la quantité totale d'anatoxine diphtérique conjuguée présente dans une dose vaccinale, est de 40 à 130 µg; et
*(4)* la quantité totale d'anatoxine diphtérique conjuguée présente dans une dose vaccinale est suffisante pour induire une protection contre les infections à *Corynebactérium diphteriae*.

Dans un mode de réalisation de l'invention, la quantité totale d'anatoxine diphtérique conjuguée présente dans une dose vaccinale est de 40 à 85 µg.

Dans un autre mode de réalisation, « p » est entre 4 et 15.

Dans un autre mode de réalisation particulier, « p » est égal à 11 et les « p » polysaccharides capsulaires des « p » conjugués polysaccharidiques sont les polysaccharides des sérotypes 1, 3, 4, 5, 6B, 7F, 9V, 14, 18C, 19F et 23F.

L'invention concerne enfin l'utilisation d'une composition comprenant « n » conjugués polysaccharidiques constitués individuellement d'un polysaccharide capsulaire de *Streptococcus pneumoniae* couplé à l'anatoxine tétanique et « p » conjugués polysaccharidiques constitués individuellement d'un polysaccharide capsulaire de *Streptococcus pneumoniae* couplé à l'anatoxine diphtérique, pour la fabrication d'un vaccin protégeant contre les infections à *Clostridium tetani* et à *Corynebactérium diphteriae ;* composition dans laquelle :
*(1)* les « n » polysaccharides capsulaires du groupe des « n » conjugués polysaccharidiques sont tous différents entre eux et les « p » polysaccharides capsulaires du groupe des « p » conjugués polysaccharidiques sont tous différents entre eux,
*(2)* « n » est supérieur ou égal à 2 et inférieur ou égal à 23,
*(3)* « p » est supérieur ou égal à 2 et inférieur ou égal à 15,
*(4)* la quantité totale d'anatoxine tétanique conjuguée présente dans une dose vaccinale est de 6 à 40 µg,
*(5)* la quantité totale d'anatoxine diphtérique conjuguée présente dans une dose vaccinale est de 40 à 130 µg; et
*(6)* les quantités totales d'anatoxine tétanique et diphtérique conjugués présentes dans une dose vaccinale sont suffisantes pour induire une protection contre les infections à *Clostridium tetani* et à *Corynebactérium diphteriae*.

Dans un mode de réalisation de l'invention, les « n » polysaccharides capsulaires sont identiques au « p » polysaccharides capsulaires.

Dans un autre mode de réalisation, les « n » polysaccharides capsulaires sont différents ou partiellement différents des « p » polysaccharides capsulaires.

Dans encore un autre mode de réalisation, « n » est entre 2 et 9, « p »est entre 2 et 11-n, les « n » polysaccharides capsulaires sont tous différents des « p » polysaccharides capsulaires et sont choisis dans le groupe des polysaccharides capsulaires des sérotypes 1, 3, 4, 5, 6B, 7F, 9V, 14, 18C, 19F et 23F.

Enfin, dans un mode de réalisation très particulier, « n » est égal à 7, « p » est égal à 4, les « n » polysaccharides capsulaires sont les polysaccharides capsulaires des sérotypes 1, 4, 5, 7F, 9V, 19F et 23F, et les « p » polysaccharides capsulaires sont les polysaccharides capsulaires des sérotypes 3, 6B, 14 et 18C.

### Description détaillée de l'invention

Dans le contexte de la présente invention, différents termes employés sont ci-après définis:
Par "polysaccharide", on entend un polymère comprenant un enchaînement de plusieurs molécules d'oses identiques ou différentes réunies entre elles par des liaisons de covalence. Par ce terme on englobe aussi celui de polyoside et d'oligoside.
Par "anatoxine", on entend une toxine détoxifiée chimiquement ou par génie génétique (par délétion, substitution ou insertion d'un ou plusieurs nucléotides), induisant une réponse immune capable de neutraliser le pouvoir pathogène de la toxine naturelle.
Par "conjugué polysaccharidique ou polysaccharide conjugué", on entend un polysaccharide couplé, soit à l'anatoxine tétanique, soit à l'anatoxine diphtérique au moyen d'une ou plusieurs liaisons covalentes. D'après cette définition, on exclut tout polysaccharide qui est conjugué à la fois à l'anatoxine tétanique et à l'anatoxine diphtérique.
Par «dose de vaccin» ou «dose vaccinale», on entend la quantité de la composition selon l'invention qui est administrée chez l'homme en une fois.
Par "la quantité totale d'anatoxine conjuguée présente dans une dose de vaccin, est suffisante pour induire une protection contre les infections à *Clostridium tetani* et/ou à *Corynebacterium diphteriae*" on entend la quantité totale d'anatoxine tétanique conjuguée contenue dans une dose de vaccin ou la quantité totale d'anatoxine diphtérique conjuguée contenue dans une demi dose de vaccin, qui lorsqu'elle est injectée, en une seule fois chez le cobaye sous la forme d'une composition selon l'invention, lui donne une chance de survie d'au moins 80% à 10 jours après un test d'épreuve qui contient 10 doses minimum mortelles (DMM) de toxine tétanique ou 10 DMM de toxine diphtérique. A titre d'illustration, si un sujet sain reçoit 3 doses d'un vaccin à intervalles réguliers (dans le cadre d'une primo vaccination par exemple) fabriqué à partir d'une composition utile aux fins de la présente invention dans laquelle une dose vaccinale contient au total 10µg d'anatoxine tétanique conjuguée et 60µg d'anatoxine diphtérique conjuguée, la quantité d'anatoxine tétanique conjuguée sera considérée comme protectrice vis à vis de *Clostridium tetani* si 80% des cobayes, immunisés avec la même composition et recevant chacun l'équivalent de 10µg d'anatoxine tétanique conjuguée, survivent à une épreuve de 10 DMM de toxine tétanique. De même, la quantité d'anatoxine diphtérique conjuguée sera considérée comme protectrice vis à vis de *Corynebacterium diphteriae* si 80% des cobayes, immunisés avec la même composition et recevant chacun l'équivalent de 30µg d'anatoxine diphtérique conjuguée, survivent à une épreuve de 10 DMM de toxine diphtérique. Pour les détails pratiques concernant le test de protection chez le cobaye, on pourra se référer à l'exemple 2. On observe une bonne correspondance entre les résultats des tests de protection pratiqués chez le cobaye et les taux de protection obtenus chez l'homme, notamment chez le nourrisson. Lorsque le vaccin est adjuvé avec un gel d'alun, on considère que la quantité d'anatoxine conjuguée présente dans une dose de vaccin adjuvé avec un gel d'alun est suffisante pour induire une protection contre les infections à *Clostridium tetani* et/ou à *Corynebacterium diphteriae* lorsque l'administration d'une demi dose de ce vaccin à chaque cobaye entraîne la production d'un immunserum dont le(s) titre(s) en anticorps neutralisants antitétaniques et/ou antidiphtériques, déterminé(s) selon les conditions détaillées de l'exemple 3, sont supérieurs à 2 UI/ml.
Par "sérotype ou sérogroupe" on entend une souche d'une espèce bactérienne définie par la structure chimique du polysaccharide capsulaire ou au moyen de l'immunsérum spécifique du polysaccharide capsulaire.

L'invention concerne l'utilisation d'une combinaison d'au moins deux polysaccharides de *Streptococcus pneumoniae* couplés à l'anatoxine tétanique et/ou d'au moins deux polysaccharides de *Streptococcus pneumoniae* couplés à l'anatoxine diphtérique dans une préparation vaccinale pour induire une immunité protectrice vis à vis de *Clostridium tetani* et/ou de *Corynebacterium diphteriae* sans qu'il soit nécessaire d'y ajouter en complément une quantité significative d'anatoxine tétanique et/ou diphtérique libre.

Une composition selon l'invention n'a pas besoin d'être associée à de l'anatoxine tétanique et/ou diphtérique existant sous une forme non-conjuguée ou sous une forme conjuguée autre que celle qui est l'objet de la présente invention au moment de son administration pour produire son effet protecteur à l'égard du tétanos et/ou de la diphtérie. Une composition selon l'invention contribue ainsi à diminuer la charge antigénique globale vaccinale administrée dans la mesure où elle induit également une immunité protectrice vis à vis des différentes souches de *Streptococcus pneumoniae* exprimant à leur surface les polysaccharides correspondant à ceux de la combinaison de conjugués. Cette utilisation a fait l'objet de la demande WO 98/51339.

L'art antérieur enseigne que la conjugaison d'une molécule porteuse, comme l'anatoxine tétanique ou diphtérique, à un polysaccharide ou un oligosaccharide entraîne une perte d'immunogénicité de la molécule porteuse résultant du masquage des épitopes inducteurs d'anticorps neutralisants ou inducteurs d'une immunité protectrice. En conséquence il faut augmenter la quantité de molécule porteuse dans le conjugué pour obtenir une immunité protectrice équivalente à celle du porteur libre. Schneerson R. et al. dans Infection and Immunity (1986); 52, 519-528) a rapporté des quantités d'anatoxine tétanique nécessaires comprises entre 84µg et 250µg dans le conjugué polysaccharidique du pneumocoque de sérotype 6B pour observer une réponse immunitaire contre l'anatoxine tétanique chez l'homme. D'une façon surprenante, lorsque, au moins deux polysaccharides différents du pneumocoque sont conjugués à l'anatoxine tétanique, la quantité totale de molécule porteuse nécessaire pour induire une immunité protectrice est nettement plus faible que celle présente dans une composition contenant un seul conjugué polysaccharidique, comme il a été mentionné par Schneerson R et al. Ainsi, la quantité totale d'anatoxine tétanique conjuguée comprise dans une combinaison d'au moins deux conjugués polysaccharidiques différents selon l'invention, de façon préférée dans une combinaison de 4 conjugués polysaccharidiques différents et de façon encore plus préférée dans une combinaison de 7 à 11 conjugués polysaccharidiques différents n'a pas besoin de dépasser 40µg pour induire une immunité protectrice contre le tétanos. D'une façon encore plus surprenante cette quantité totale maximale nécessaire est plus faible que la dose totale de 54µg d'anatoxine tétanique incluse dans le vaccin pentavalent DTP-IPV-PRP-T(diphtérie, tétanos, coqueluche, polio, conjugué haemophilus) déjà commercialisé (30µg d'anatoxine existe sous forme non conjuguée et 24µg existe sous forme conjuguée au polysaccharide capsulaire d*'haemophilus influenzae*). L'association de plusieurs polysaccharides différents du pneumocoque conjugués à une même protéine porteuse diminue les effets négatifs sur l'immunogénicité du porteur observé lors de l'utilisation d'un seul conjugué et favorise donc le développement d'une immunité protectrice vis à vis du porteur.

Pour réaliser la conjugaison de l'anatoxine tétanique aux polysaccharides du pneumocoque on en choisit au moins deux parmi les 23 sérotypes 1, 2, 3, 4, 5, 6B, 7F, 8, 9N, 9V, 10A, 11A, 12F, 14, 15B, 17F, 18C, 19F, 19A, 20, 22F, 23F, 33F, mais préférentiellement au moins deux parmi les 11 sérotypes 1, 3, 4, 5, 6B, 7F, 9V, 14, 18C, 19F, et 23F. Ainsi une composition de conjugués polysaccharidiques induisant une immunité protectrice vis à vis du tétanos contient de préférence entre 2 et 11 conjugués différents, de préférence de 4 à 11 conjugués différents, et de façon encore plus préférée de 7 à 11 conjugués différents, la quantité totale d'anatoxine tétanique conjuguée dans une dose vaccinale allant de 6 à 40µg, de préférence de 10µg à 25µg. De façon toute préférée, une composition selon l'invention est constituée de 11 conjugués polysaccharides capsulaires correspondant aux sérotypes 1, 3, 4, 5, 6B, 7F, 9V, 14, 18C, 19F et 23F du pneumocoque et renferme une quantité totale d'anatoxine tétanique conjuguée comprise entre 10µg et 25µg par dose vaccinale. Cette dose est d'une façon très surprenante équivalente à celle contenue par exemple dans un vaccin classique comme le DTP (diphtérie, tétanos, polio) dans lequel l'anatoxine tétanique existe uniquement sous forme libre à une dose comprise entre 15 et 30µg ou encore comprise entre 5Lf et 10Lf (méthode de quantification des anatoxines en unités de floculation). Puisque la protection contre le tétanos est obtenue par injection d'une dose de vaccin comprenant 11 conjugués polysaccharidiques dont la quantité totale d'anatoxine tétanique conjuguée n'est pas plus importante que celle contenue dans le vaccin DTP cela indique que les conjugués polysaccharidiques coopèrent entre eux, d'une façon surprenante, pour annuler les effets négatifs de la conjugaison sur l'immunogénicité du porteur. Par ailleurs, les conjugués polysaccharidiques couplés à l'anatoxine tétanique selon l'invention induisent une immunité protectrice vis à vis des sérotypes/sérogroupes correspondants du pneumocoque.

Pour réaliser la conjugaison d'un polysaccharide de *Streptococcus pneumoniae* à l'anatoxine diphtérique il faut prendre en compte le fait que la quantité d'anatoxine diphtérique nécessaire pour obtenir une protection contre la diphtérie est environ entre 2 et 4 fois plus importante que celle requise pour l'anatoxine tétanique. Pour ne pas observer d'interférences négatives sur la réponse immune vis à vis des polysaccharides du pneumocoque, la quantité totale d'anatoxine diphtérique conjuguée par dose vaccinale est comprise entre 40 et 130µg sans toutefois excéder 130µg, et de préférence entre 40 et 85µg. Pour cette même raison, une composition de polysaccharides de *Streptococcus pneumoniae* conjugués à l'anatoxine diphtérique peut contenir entre 2 et 15 conjugués différents sans toutefois excéder la valeur de 15 conjugués différents, mais contient de préférence entre 4 et 15 conjugués différents et de façon encore plus préférée entre 7 et 15 conjugués différents. Pour la préparation des conjugués diphtériques, on peut choisir entre 2 et 15 polysaccharides capsulaires différents parmi les 23 identifiés. De façon toute préférée, une composition selon l'invention est constituée de 11 conjugués polysaccharides capsulaires correspondant aux sérotypes 1, 3, 4, 5, 6B, 7F, 9V, 14, 18C, 19F, et 23F et renferme une quantité totale d'anatoxine diphtérique conjuguée comprise entre 40µg et 85 µg par dose vaccinale. D'une façon très surprenante, la quantité totale d'anatoxine diphtérique conjuguée dans ce type de composition est équivalente à celle contenue par exemple dans un vaccin classique comme le DTP (diphtérie, tétanos, polio) dans lequel l'anatoxine diphtérique existe uniquement sous forme libre à une dose comprise entre 45 et 90µg ou encore comprise entre 15Lf et 30Lf. Les conjugués de la composition qui sont couplés à l'anatoxine diphtérique coopèrent entre eux, d'une façon inexpliquée pour annuler les effets négatifs de la conjugaison sur l'immunogénicité du porteur notamment. Par ailleurs, une composition de polysaccharides de *Streptococcus pneumoniae* couplés à l'anatoxine diphtérique selon l'invention induit une immunité protectrice vis à vis des différentes souches de *Streptococcus pneumoniae* exprimant à leur surface les polysaccharides correspondant à ceux de la composition de conjugués.

Une composition de polysaccharides conjugués selon l'invention peut également comprendre au moins deux polysaccharides conjugués à l'anatoxine tétanique et au moins deux polysaccharides conjugués à l'anatoxine diphtérique pour induire une immunité protectrice vis à vis de *Clostridium tetani* et vis à vis de *Corynebacterium diphteriae*. Les polysaccharides peuvent être tous différents et choisis parmi ceux correspondant aux 23 sérotypes du pneumocoque. Dans ce type de composition, le nombre total p de conjugués à l'anatoxine diphtérique peut varier entre 2 et 15 pour les raisons invoquées plus haut, le nombre total n de conjugués à l'anatoxine tétanique pouvant varier quant à lui entre 2 et 23-p, les quantités totales d'anatoxine tétanique et d'anatoxine diphtérique conjuguées contenues dans une dose vaccinale étant comprises respectivement entre 6 et 40µg et entre 40 et 130µg. De façon préférentielle, on restreint le choix des polysaccharides aux sérotypes 1, 3, 4, 5, 6B, 7F, 9V, 14, 18C, 19F, et 23F du pneumocoque. Dans ce type de composition, le nombre total n de polysaccharides conjugués à l'anatoxine tétanique varie de préférence entre 2 et 9 et de façon encore plus préférée entre 4 et 7, le nombre total p de conjugués à l'anatoxine diphtérique varie de préférence entre 2 et 11-n et de façon encore plus préférée entre 4 et 7, les quantités totales d'anatoxine tétanique et d'anatoxine diphtérique conjugués étant respectivement comprises de façon préférentielle entre 10 et 25 µg et entre 40 µg et 85µg par dose vaccinale. D'une manière tout particulièrement préférée, une composition de conjugués selon l'invention comprend 7 polysaccharides conjugués à l'anatoxine tétanique correspondant aux sérotypes 1, 4, 5, 7V, 9V, 19F, et 23F et 4 polysaccharides conjugués à l'anatoxine diphtérique correspondant aux sérotypes 3, 6B, 14 et 18C, les quantités totales d'anatoxine tétanique et diphtérique conjuguées contenues dans une dose vaccinale étant préférentiellement comprises entre 10 et 15µg et entre 40µg et 65µg.

Un ou plusieurs polysaccharides dans une composition selon l'invention peut(vent) être utilisé à la fois pour la préparation du conjugué à l'anatoxine tétanique et du conjugué à l'anatoxine diphtérique pour autant que la composition respecte les conditions de l'invention c'est à dire qu'elle contienne au moins deux conjugués à l'anatoxine tétanique différents et au moins deux conjugués à l'anatoxine diphtérique différents. Le nombre total de conjugués polysaccharides couplés à l'anatoxine tétanique et de conjugués polysaccharides couplés à l'anatoxine diphtérique peut être au maximum de 38, 23 conjugués étant couplés à l'anatoxine tétanique et 15 conjugués couplés à l'anatoxine diphtérique. Dans un aspect particulier, tous les polysaccharides servant à la préparation des conjugués à l'anatoxine tétanique servent également à la préparation des conjugués à l'anatoxine diphtérique. Dans ce cas la composition vaccinale contient le même nombre de conjugués à l'anatoxine tétanique et de conjugués à l'anatoxine diphtérique, ce nombre étant compris entre 2 et 15 car le nombre de conjugués à l'anatoxine diphtérique ne peut pas dépasser 15 pour des raisons de charge antigénique globale.

Une composition comprenant la combinaison de polysaccharides conjugués à l'anatoxine tétanique et de polysaccharides conjugués à l'anatoxine diphtérique induit une immunité protectrice vis à vis de *Clostridium tetani* et de *Corynebacterium diphteriae*. Par ailleurs, la combinaison, par le biais des polysaccharides présents, contribue également à l'induction d'une immunité protectrice vis à vis des sérotypes correspondants du pneumocoque. La combinaison des polysaccharides conjugués à l'anatoxine tétanique et des polysaccharides conjugués à l'anatoxine diphtériques n'entraîne pas l'apparition d'interférences négatives sur le développement de la réponse immune aux différents polysaccharides, dans la mesure où les conditions sur n et p sont respectées.

Les polysaccharides peuvent être avantageusement extraits des différentes souches de *Streptococcus pneumoniae* selon des procédés conventionnels et purifiés de même. Ces polysaccharides peuvent être utilisés sous forme brute après extraction/purification. Ou bien encore ils peuvent être fragmentés afin d'obtenir des polysaccharides de poids moléculaires moyens inférieurs à ceux des polysaccharides d'origine. Un procédé de fragmentation particulièrement avantageux est décrit dans WO 93/07178.

Un conjugué dans lequel un polysaccharide est couplé par liaison covalente à une protéine peut être obtenu selon des procédés conventionnels bien connus de l'homme de l'art. Il peut être fait usage de "linker" ou de "spacer" afin d'assurer la conjugaison. Selon le mode de conjugaison mis en oeuvre, le conjugué qui en résulte peut être un conjugué dans lequel le polysaccharide est lié à la protéine par une seule fonction chimique (type soleil ou néoglyconjugué) ou par plusieurs fonctions (type pelote). Il est à la portée de l'homme de l'art de déterminer le mode de conjugaison le plus approprié en fonction de la nature du polysaccharide et plus particulièrement des groupements chimiques portés par le polysaccharide qui peuvent être mis en jeu au cours de la réaction de conjugaison.

Ci-après on présente à titre d'exemple la préparation de différentes compositions selon l'invention, les polysaccharides choisis étant issus des sérotypes 1, 3, 4, 5, 6B, 7F, 9V, 14, 18C, 19F, et 23F. Les polysaccharides issus de ces sérotypes ont été fragmentés selon le procédé décrit dans WO 93/07178 et sont couplés à l'anatoxine tétanique (sauf le polysaccharide de type 1) selon le procédé de conjugaison décrit dans WO 93/07178. Brièvement, un polysaccharide est soumis à une amination réductrice en présence de cyanoborohydrure de sodium afin de lier une molécule de diaminohexane à un groupe réducteur terminal. Puis le polysaccharide ainsi dérivé est activé par un groupe succinimide en utilisant du disuccinimidyl suberate (DSS). Le polysaccharide ainsi activé est mis à réagir directement avec la protéine porteuse. Le polysaccharide de sérotype 1 est couplé à l'anatoxine diphtérique ou à l'anatoxine tétanique selon le procédé de conjugaison décrit dans le brevet US n°5,204,098. Les conditions expérimentales ont été contrôlées de manière à ce que l'on obtienne des conjugués dans lesquels la quantité de protéine représente entre 1 et 4 fois, de préférence 2 fois, la quantité de polysaccharide. Ainsi pour un conjugué polysaccharidique couplé à l'anatoxine tétanique, 1µg d'un polysaccharide particulier peut être couplé à environ 2µg d'anatoxine tétanique. La quantité d'anatoxine tétanique résiduelle non couplée est très faible à l'issue de chaque réaction de conjugaison. On complète au besoin l'élimination de l'anatoxine libre résiduelle par une dialyse ou une ultrafiltration. La composition de polysaccharides conjugués à l'anatoxine tétanique est obtenue en mélangeant les différents conjugués entre eux de telle façon que la quantité de polysaccharide contenue dans chaque conjugué soit au moins celle nécessaire pour observer une immunité protectrice vis à vis du sérotype correspondant et que la quantité totale en anatoxine tétanique conjuguée comprise dans une dose vaccinale soit comprise entre 10 et 40µg et de façon préférentielle comprise entre 10 et 25µg. On contrôle également par électrophorèse capillaire ou par chromatographie que la quantité résiduelle d'anatoxine tétanique non conjuguée dans une composition selon l'invention représente moins de 5% de la quantité totale d'anatoxine tétanique conjuguée. La composition décrite dans l'exemple 1.1 est préparée selon les techniques opératoires susmentionnées.

Les polysaccharides couplés à l'anatoxine diphtérique l'ont été comme suit: des groupes hydrazide sont incorporés sur le polysaccharide en faisant réagir le polysaccharide avec un excès d'acide adipique dihydrazide (ADH) en présence d'éthyl diméthylamino propyl carbodiimide (EDAC) et de cyanoborohydrure de sodium (pour tous les types sauf le 3) ou simplement en présence de cyanoborohydrure de sodium (pour le type 3). Le polysaccharide ainsi dérivé est mis à réagir avec la protéine porteuse en présence d'EDAC. Les conditions expérimentales ont été contrôlées de manière à ce que l'on obtienne des conjugués dans lesquels la quantité de protéine représente entre 1 et 4 fois la quantité de polysaccharide. La quantité d'anatoxine diphtérique résiduelle non couplée est très faible à l'issue de chaque réaction de conjugaison. On complète au besoin l'élimination de l'anatoxine libre résiduelle par une dialyse ou une ultrafiltration. La composition de polysaccharides conjugués à l'anatoxine diphtérique est obtenue en mélangeant les différents conjugués entre eux de telle façon que la quantité de polysaccharide contenue dans chaque conjugué soit au moins celle nécessaire pour observer une immunité protectrice vis à vis du sérotype correspondant et que la quantité totale en anatoxine diphtérique conjuguée comprise dans une dose vaccinale soit comprise entre 40 et 130µg et de façon préférentielle comprise entre 40 et 85µg. On contrôle également par électrophorèse capillaire ou par chromatographie liquide haute performance que la quantité résiduelle d'anatoxine diphtérique non conjuguée dans une composition selon l'invention représente moins de 5% de la quantité totale d'anatoxine diphtérique conjuguée.

L'anatoxine tétanique et l'anatoxine diphtérique ont été préparées par détoxification au formaldéhyde à partir des toxines extraites respectivement de *Corynebacterium diphteriae* et de *Clostridium tetani* bien connue de l'homme de métier. L'anatoxine diphtérique peut être également un mutant non toxique de la toxine diphtérique comme par exemple le composé CRM197. Les anatoxines tétaniques et diphtériques utilisées pour la préparation des conjugués polysaccharidiques ont un taux de pureté supérieur à 90%.

Une composition selon l'invention qui comprend à la fois des conjugués polysaccharidiques couplés à l'anatoxine tétanique et des conjugués polysaccaridiques couplés à l'anatoxine diphtérique est fabriquée en mélangeant les différents conjugués polysaccharidiques qui ont été préparés individuellement et en prenant en compte que les conjugués polysaccharidiques couplés à l'anatoxine diphtérique sont proportionnellement en quantités plus importantes que les conjugués polysaccharidiques couplés à l'anatoxine tétanique. Ainsi, dans une composition qui comporte autant de conjugués polysaccharidiques couplés à l'anatoxine diphtérique, que de conjugués couplés à l'anatoxine tétanique le poids total des conjugués couplés à l'anatoxine diphtérique est en moyenne entre 3 et 6 fois plus important que le poids total des conjugués couplés à l'anatoxine tétanique. C'est ce qu'on observe dans la composition décrite dans l'exemple 1.2.

Une composition selon l'invention peut être formulée avec un diluant ou un support acceptable d'un point de vue pharmaceutique e.g., un hydroxyde d'aluminium, un phosphate d'aluminium ou un hydroxyphosphate d'aluminium, et le cas échéant un excipient de lyophilisation. En général, ces produits peuvent être sélectionnés en fonction du mode et de la voie d'administration et selon les pratiques pharmaceutiques standard. Les diluants appropriés ainsi que ce qui est indispensable à l'élaboration d'une composition pharmaceutique sont décrits dans *Remington's Pharmaceutical Sciences,* un livre de référence standard dans ce domaine.

Une composition selon l'invention contient avantageusement un tampon phosphate et du chlorure de sodium et peut être adjuvanté par de l'hydroxyde d'aluminium. On peut également utiliser un conservateur tel que le phenoxyéthanolformol. Une dose vaccinale peut être préparée sous un volume de 0,1ml à 2ml, et de façon préférée sous un volume de 0,5ml et contenir 0,475mg d'ion PO₄²⁻, 4,5mg de chlorure de sodium et éventuellement 300 µg d'ions Al³⁺.

### Exemple 1: Composition de polysaccharides capsulaires de Streptococcus pneumoniae conjugués à l'anatoxine tétanique (ATT)

1.1: Composition tétravalente constituée des polysaccharides capsulaires des sérotypes 23F, 14, 19, 6B conjugués à l'anatoxine tétanique et correspondant à une dose vaccinale humaine.

| Serotype | Quantité de polysaccharide (µg) | Quantité d'ATT (µg) |
|---|---|---|
| 23F | 1 | 1,5 |
| 14 | 1 | 2 |
| 19F | 1 | 1,5 |
| 6B | 1 | 1,5 |
| La quantité totale de polysaccharide conjugué contenue dans une dose vaccinale humaine est de 4µg | | |
| La quantité totale d'ATT conjuguée contenue dans une dose vaccinale humaine est de 6,5µg | | |
| ATT= anatoxine tétanique | | |
| Le volume total d'une dose vaccinale est de 0,5ml | | |

1.2: Composition à 11 valences constituée des polysaccharides capsulaires des sérotypes 1, 4, 5, 7F, 9V, 19F et 23F conjugués à l'anatoxine tétanique et des polysaccharides capsulaires des sérotypes 3, 6B, 14, 18C conjugués à l'anatoxine diphtérique et correspondant à une dose vaccinale humaine.

| serotype | Quantité de polysaccharide (en µg) | Quantité d'ATT (en µg) | Quantité de DT (en µg) |
|---|---|---|---|
| 1 | 1 | 2,7 | |
| 3 | 3 | | 15 |
| 4 | 1 | 1,7 | |
| 5 | 1 | 1 | |
| 6B | 10 | | 31 |
| 7F | 1 | 1,3 | |
| 9V | 1 | 1,6 | |
| 14 | 3 | | 8 |
| 18C | 3 | | 6 |
| 19F | 1 | 2,5 | |
| 23F | 1 | 1,2 | |
| La quantité totale de polysaccharide conjugué contenue dans une dose vaccinale est de 26 µg | | | |
| La quantité totale de DT conjuguée contenue dans une dose vaccinale est de 60 µg | | | |
| La quantité totale d'ATT conjuguée contenue dans une dose vaccinale est de 12 µg | | | |
| DT= Anatoxine diphtérique | | | |
| Le volume total d'une dose vaccinale est de 0,5 ml | | | |

### Exemple 2: Protection des cobayes vis à vis du tétanos et/ou de la diphtérie à l'aide de diverses compositions de polysaccharides capsulaires de Streptococcus pneumoniae couplés à l'anatoxine tétanique (ATT) et /ou à l'anatoxine diphtérique (DT)

Les compositions de polysaccharides capsulaires de *Streptococcus pneumoniae* couplés à l'anatoxine tétanique (ATT) et /ou à l'anatoxine diphtérique (DT) décrites dans l'exemple 1, ont été testées chez le Cobaye pour leur capacité à protéger ces animaux vis à vis du tétanos ou de la diphtérie. On a testé aussi des compositions à 11 valences de polysaccharides couplés uniquement à l'anatoxine tétanique, dont celle constituée des polysaccharides capsulaires 1, 3, 4, 5, 6B, 7F, 9V, 14, 18C, 19F et 23F. Les compositions correspondantes à 11 valences de polysaccharides couplés uniquement à l'anatoxine diphtérique ont également été étudiés. On a testé une composition à 15 valences constituée des polysaccharides capsulaires des sérotypes 1, 4, 5, 6B, 7F, 9V, 18C, 19F et 23F conjugués à l'anatoxine tétanique et des polysaccharides capsulaires des sérotypes 3, 6B, 9V, 14, 18C, 23F conjugués à l'anatoxine diphtérique. Enfin on a testé une composition à 15 valences constituée des polysaccharides capsulaires des sérotypes 1, 3, 4, 5, 6B, 7F, 8, 9V, 12F, 14, 15, 18C, 19F, 22F, 23F qui sont tous couplés à l'anatoxine tétanique et la même composition de polysaccharides qui sont couplés à l'anatoxine diphtérique. Toutes ces compositions étaient en milieu liquide. L'excipient utilisé contenait un tampon phosphate et du chlorure de sodium.

On a constitué des groupes de 10 cobayes pour chaque composition testée. Les animaux ont reçu par voie sous cutanée, 1/3 d'une dose vaccinale humaine totale lorsqu'il était prévu ensuite de les éprouver avec la toxine tétanique. La dose vaccinale humaine totale est la somme des doses vaccinales que le sujet reçoit lors de la primo vaccination. Dans un schéma classique de primo vaccination à 3 injections, la dose vaccinale humaine totale est la somme des 3 doses vaccinales. Chaque cobaye a reçu une injection d'une dose vaccinale humaine sous 0,5 ml. Lorsqu'il était prévu d'éprouver les cobayes avec la toxine diphtérique, ceux ci ont reçu 1/6 de la dose vaccinale humaine totale, ce qui correspondait, pour un protocole de primo vaccination à 3 injections, à l'injection d'une demi dose vaccinale humaine sous 0,25 ml. Par exemple, cela correspondait à l'administration d'une dose vaccinale de la composition à 11 valences telle que décrite dans l'exemple 1.2, pour les animaux éprouvés avec la toxine tétanique et à l'administration d'une demi dose vaccinale de la composition à 11 valences telle que décrite dans l'exemple 1.2 pour les animaux éprouvés à la toxine diphtérique.

30 jours après l'injection des compositions vaccinales testées, les animaux immunisés ont ensuite été éprouvés par injection sous cutanée dans chaque animal de 10 doses minimum mortelles (DMM) de toxine tétanique ou de 10 DMM de toxine diphtérique. En parallèle, deux groupes témoins d'animaux non immunisés constitués de 2 et 3 cobayes ont été éprouvés respectivement avec 1 DMM de toxine tétanique et 1 DMM de toxine diphtérique. Ces groupes témoins ont servi à valider la DMM des 2 toxines sur les animaux témoins qui devaient tous mourir dans les 96 heures qui ont suivi l'épreuve. On a dénombré également les animaux morts dans les autres groupes de cobayes immunisés éprouvés. La combinaison vaccinale testée est considérée comme protectrice vis à vis du tétanos et/ou de la diphtérie si un taux minimum de 80% de survie est constatée au bout de 10 jours. Les études de survie réalisées chez les cobayes immunisés avec les différentes compositions de conjugués polysaccharidiques mentionnées, ont révélé que les taux de survie étaient tous supérieurs à 80% après épreuve avec la toxine tétanique ou diphtérique.

### Exemple 3: Etude du pouvoir protecteur d'un pool d'immun sérums obtenus à partir de cobayes immunisés avec une composition à 11 valences adjuvée avec de l'alun.

On a testé la composition telle que décrite dans l'exemple 1.2 mélangée avec un gel d'alun en utilisant un test de protection légèrement différent. La préparation du mélange est décrite dans l'exemple 4.
Selon que l'on cherche à évaluer le pouvoir protecteur de cette formulation adjuvée vis à vis d'une infection à *Clostridium tetani* ou à *Corynebacterium diphteriae*, les animaux éprouvés avec un mélange constitué d'immunsérums de cobayes et de toxine, soit tétanique soit diphtérique, sont respectivement des souris ou des cobayes. La première partie du protocole expérimental est la même dans les deux cas. Le test tel que pratiqué chez la souris tient compte des recommandations publiées par le NIH lors d'une 4^{ème} révision effectuée le 15/12/1952. Pour le test pratiqué chez le cobaye, on a tenu compte des recommandations du NIH publiée dans une 4^{ème} révision du 1/03/1947.

Dans la première commune du protocole, un groupe de cobayes a été immunisé par une demi dose vaccinale humaine totale de la composition adjuvée. Les immunsérums de chaque cobaye ont ensuite été collectés et rassemblés en un seul pool.
Pour évaluer le pouvoir protecteur de ce pool de sérum chez la souris, on a réalisé une dilution au 1/10 du pool et on a préparé un sérum étalon de référence titrant 0,1 UI/ml d'anticorps anti-tétaniques. On a réalisé ensuite un titrage d'anticorps anti-tétaniques du pool de sérum par séroneutralisation in vivo chez la souris grâce au sérum étalon de référence. On a vérifié au préalable que l'injection, sous un volume de 0,5ml, d'un mélange constitué de 0,01UI du sérum étalon et d'une dose létale 100 de toxine tétanique dans la veine caudale de chaque souris provoquait la mort en 96 heures de toutes les souris qui ont reçu ce mélange. Un pool de sérum est considéré comme protecteur, et par voie de conséquence la formulation adjuvée, s'il a un titre > 2UI/ml.

Pour évaluer le pouvoir protecteur du pool de sérum chez le cobaye, on a réalisé un titrage d'anticorps anti-diphtériques du pool de sérum par séroneutralisation in vivo chez le cobaye grâce à un sérum anti-diphtérique de référence qui titrait 6UI/ml. On a vérifié au préalable que l'injection sous-cutanée, sous un volume de 3 ml, d'un mélange qui contenait 1U du sérum antidiphtérique de référence et une dose létale 100 de toxine diphtérique (1DL100) tuait en 96 heures tous les cobayes qui ont reçu ce mélange. On a évalué ensuite le titre du pool de sérum qui doit être supérieur à 2UI/ml pour être protecteur.
Le pool de sérums obtenue à partir de la formulation adjuvée testée avait un titre en anticorps anti-tétaniques et anti-diphtériques supérieur à 2UI/ml, évalué dans les tests de séroneutralisation pratiqués *in vivo* chez la souris ou le cobaye ce qui atteste du pouvoir protecteur de cette formulation.

### Exemple 4: Etude de la réponse immune vis à vis de l'anatoxine tétanique et/ou de l'anatoxine diphtérique après injection d'une composition de polysaccharides capsulaires de Streptococcus pneumoniae couplés à l'anatoxine tétanique (ATT) et /ou à l'anatoxine diphtérique (DT)

Différentes compositions de polysaccharides capsulaires de *Streptococcus pneumoniae* couplés à l'anatoxine tétanique (ATT) et /ou à l'anatoxine diphtérique (TD) ont été testées pour leur capacité à induire une réponse anticorps spécifique dirigée contre l'anatoxine tétanique et/ ou l'anatoxine diphtérique. Dans ces études on a inclus notamment les combinaisons mentionnées dans l'exemple 2.

### Chez le singe

2 groupes de deux singes macaques (*Macaca fascicularis*) ont reçu, sous un volume de 0,65ml, par voie intra musculaire, à 4 semaines d'intervalle, 2 injections d'une combinaison à 11 valences décrite dans l'exemple 1.2 adjuvée ou non avec un gel d'alun. La dose administrée à chaque injection correspondait à une dose vaccinale humaine (le diluant utilisé était un tampon phosphate 10mM, pH 6,8, réalisé dans du chlorure de sodium à 0,9%). La combinaison adjuvée a été préparée par mélange d'une dose vaccinale humaine avec un volume de gel d'alumine contenant l'équivalent de 300µg d'Al³⁺. Des prélèvements sanguins ont été effectués le jour de la première immunisation (J0), le jour de la deuxième immunisation (J28) et 4 semaines après la deuxième immunisation (J56) pour analyser le contenu en anticorps spécifiques dirigés contre l'anatoxine tétanique et l'anatoxine diphtérique. Le dosage des anticorps spécifiques de type IgG a été réalisé par ELISA. De façon classique et connue, le dosage a été réalisé en sensibilisant des microplaques à l'aide d'une solution d'anatoxine tétanique ou diphtérique purifiée diluée, suivie d'une phase de saturation des plaques. On a réalisé ensuite, pour chaque sérum de singe testé une gamme de dilution que l'on a déposé dans les micropuits. En parallèle, on a préparé une gamme de référence à partir d'un pool d'immunsérums humains ainsi que les contrôles de qualité (comprenant notamment des sérums anti-tétaniques et anti-diphtériques ayant des titres connus, provenant du National Institute for Biological Standard and Control). Après une nouvelle phase d'incubation, suivie de plusieurs lavages pour éliminer les anticorps non spécifiques on a déposé dans chaque puits un volume d'une solution diluée d'un anticorps monoclonal anti-IgG humaines couplé à la peroxydase qui reconnaît également les IgG de macaques. Après incubation du conjugué suivie de lavages et révélation du conjugué fixé par coloration à l'aide de l'O-phénylène diamine, on a mesuré l'intensité de la coloration de chaque puits par lecture spectrophotométrique. Les résultats ont été exprimés en unités internationales par ml (UI/ml).
Le tableau ci dessous regroupe les valeurs des taux d'anticorps spécifiques obtenus avec la combinaison à 11 valences décrites dans l'exemple 1.2 adjuvée ou non avec le gel d'alun

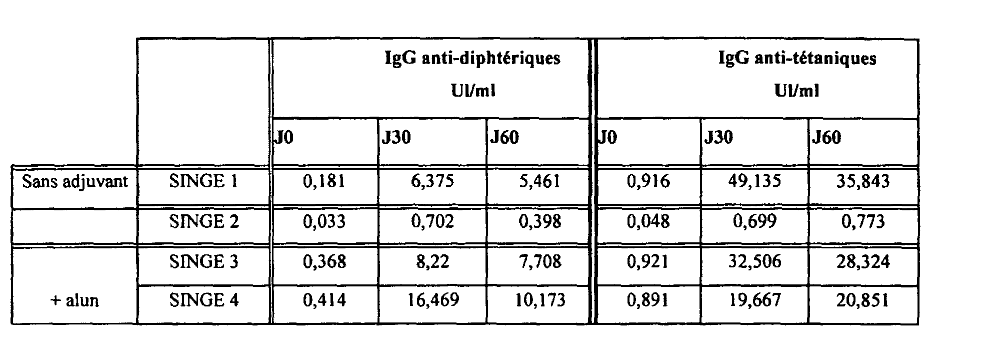

Les résultats montrent une augmentation très nette du taux d'anticorps spécifiques anti-tétaniques et anti-diphtériques. On note aussi un effet adjuvant du gel d'alun car les titres en anticorps spécifiques sont plus élevés.

### Chez l'homme

Un groupe de 12 adultes sains ont reçu 2 injections d'une dose vaccinale à 4 semaines d'intervalle d'une combinaison vaccinale tétravalente telle que décrite dans l'exemple 1.1. Des prélèvements sanguins ont été effectués avant la première immunisation (J0), le jour de la deuxième immunisation (J28) et 4 semaines après la deuxième immunisation (J56) pour analyser le contenu en anticorps spécifiques dirigés contre l'anatoxine tétanique. Le dosage des anticorps spécifiques de type IgG a été réalisé par ELISA comme décrit précédemment.
A J0 le titre moyen en anticorps spécifiques de l'anatoxine tétanique est de 5,01 UI/ml. Ce titre s'élève à 9,15 UI/ml à J28. La deuxième immunisation n'a pas d'effet sur le titre en anticorps anti-tétaniques (à J56 il est de 8,71 UI/ml). Le vaccin tétravalent provoque une élévation du titre en anticorps antitétaniques. La combinaison vaccinale induit donc une réponse immune spécifique vis à vis de la molécule porteuse. D'autres études d'immunogénicité similaires ont été réalisées chez le nourrisson, le jeune enfant avec d'autres compositions, notamment avec celles mentionnées dans l'exemple 2. Elles ont révélé également que ces combinaisons induisent des réponses immunes spécifiques dirigées contre l'anatoxine tétanique et/ou l'anatoxine diphtérique.

## Revendications

1. Utilisation d'une composition comprenant « n » conjugués polysaccharidiques constitués individuellement d'un polysaccharide capsulaire de *Streptococcus pneumoniae* couplé à l'anatoxine tétanique, pour la fabrication d'un vaccin protégeant contre les infections à *Clostridium tetani*, composition dans laquelle:
*(1)* les « n » polysaccharides capsulaires des « n » conjugués polysaccharidiques sont tous différents,
*(2)* « n » est supérieur ou égal à 2 et inférieur ou égal à 23,
*(3)* la quantité totale d'anatoxine tétanique conjuguée présente dans une dose vaccinale, est de 6 à 40 µg ; et
*(4)* la quantité totale d'anatoxine tétanique conjuguée présente dans une dose vaccinale est suffisante pour induire une protection contre les infections à *Clostridium tetani*.

2. Utilisation selon la revendication 1, d'une composition dans laquelle la quantité totale d'anatoxine tétanique conjuguée présente dans une dose vaccinale est de 10 à 25µg.

3. Utilisation selon la revendication 1 ou 2, d'une composition dans laquelle « n » est entre 4 et 11.

4. Utilisation selon la revendication 3, d'une composition dans laquelle « n » est égal à 11 et dans laquelle les « n » polysaccharides capsulaires des « n » conjugués polysaccharidiques sont les polysaccharides des sérotypes 1, 3, 4, 5, 6B, 7F, 9V, 14, 18C, 19F et 23F.

5. Utilisation d'une composition comprenant « p » conjugués polysaccharidiques constitués individuellement d'un polysaccharide capsulaire de *Streptococcus pneumoniae* couplé à l'anatoxine diphtérique, pour la fabrication d'un vaccin protégeant contre les infections à *Corynebactérium diphteriae*, composition dans laquelle:
*(1)* les « p » polysaccharides capsulaires des « p » conjugués polysaccharidiques sont tous différents,
*(2)* « p » est supérieur ou égal à 2 et inférieur ou égal à 15,
*(3)* la quantité totale d'anatoxine diphtérique conjuguée présente dans une dose vaccinale, est de 40 à 130 µg; et
*(4)* la quantité totale d'anatoxine diphtérique conjuguée présente dans une dose vaccinale est suffisante pour induire une protection contre les infections à *Corynebactérium diphteriae*.

6. Utilisation selon la revendication 5, d'une composition dans laquelle la quantité totale d'anatoxine diphtérique conjuguée présente dans une dose vaccinale est de 40 à 85 µg.

7. Utilisation selon la revendication 5 ou 6, d'une composition dans laquelle « p » est entre 4 et 15.

8. Utilisation selon la revendication 7, d'une composition dans laquelle « p » est égal à 11 et dans laquelle les « p » polysaccharides capsulaires des « p » conjugués polysaccharidiques sont les polysaccharides des sérotypes 1, 3, 4, 5, 6B, 7F, 9V, 14, 18C, 19F et 23F.

9. Utilisation d'une composition comprenant « n » conjugués polysaccharidiques constitués individuellement d'un polysaccharide capsulaire de *Streptococcus pneumoniae* couplé à l'anatoxine tétanique et « p » conjugués polysaccharidiques constitués individuellement d'un polysaccharide capsulaire de *Streptococcus pneumoniae* couplé à l'anatoxine diphtérique, pour la fabrication d'un vaccin protégeant contre les infections à *Clostridium tetani* et à *Corynebactérium diphteriae ;* composition dans laquelle :
*(1)* les « n » polysaccharides capsulaires du groupe des « n » conjugués polysaccharidiques sont tous différents entre eux et les « p » polysaccharides capsulaires du groupe des « p » conjugués polysaccharidiques sont tous différents entre eux,
*(2)* « n » est supérieur ou égal à 2 et inférieur ou égal à 23,
*(3)* « p » est supérieur ou égal à 2 et inférieur ou égal à 15,
*(4)* la quantité totale d'anatoxine tétanique conjuguée présente dans une dose vaccinale est de 6 à 40 µg,
*(5)* la quantité totale d'anatoxine diphtérique conjuguée présente dans une dose vaccinale est de 40 à 130 µg; et
*(6)* les quantités totales d'anatoxine tétanique et diphtérique conjugués présentes dans une dose vaccinale sont suffisantes pour induire une protection contre les infections à *Clostridium tetani* et à *Corynebactérium diphteriae*.

10. Utilisation selon la revendication 9, d'une composition dans laquelle les « n » polysaccharides capsulaires sont identiques au « p» polysaccharides capsulaires.

11. Utilisation selon la revendication 9, d'une composition dans laquelle les « n » polysaccharides capsulaires sont différents ou partiellement différents des « p » polysaccharides capsulaires.

12. Utilisation selon la revendication 11, d'une composition dans laquelle :
*(1)* n est entre 2 et 9,
*(2)* p est entre 2 et 11-n,
*(3)* les « n » polysaccharides capsulaires sont tous différents des « p » polysaccharides capsulaires et sont choisis dans le groupe des polysaccharides capsulaires des sérotypes 1, 3, 4, 5, 6B, 7F, 9V, 14, 18C, 19F et 23F.

13. Utilisation selon la revendication 12, d'une composition dans laquelle :
*(1)* « n » est égal à 7,
*(2)* « p » est égal à 4,
*(3)* les « n » polysaccharides capsulaires sont les polysaccharides capsulaires des sérotypes 1, 4, 5, 7F, 9V, 19F et 23F, et
*(4)* les « p » polysaccharides capsulaires sont les polysaccharides capsulaires des sérotypes 3, 6B, 14 et 18C.

## Patentansprüche

1. Verwendung einer Zusammensetzung, die "n" Polysaccharid-Konjugate umfasst, welche jeweils aus einem an das Tetanusanatoxin gekoppelten Kapselpolysaccharid von *Streptococcus pneumoniae* bestehen, zur Herstellung eines Impfstoffs, der gegen die Infektionen mit *Clostridium tetani* schützt, wobei in der Zusammensetzung:
*(1)* die "n" Kapselpolysaccharide der "n" Polysaccharid-Konjugate alle verschieden sind,
*(2)* "n" größer oder gleich 2 und kleiner oder gleich 23 ist,
*(3)* die Gesamtmenge an konjugiertem Tetanusanatoxin, die in einer Impfdosis vorliegt, 6 bis 40 µg beträgt und
*(4)* die Gesamtmenge an konjugiertem Tetanusanatoxin, die in einer Impfdosis vorliegt, ausreicht, um einen Schutz gegen die Infektionen mit *Clostridium tetani* zu induzieren.

2. Verwendung gemäß Anspruch 1 einer Zusammensetzung, worin die Gesamtmenge an konjugiertem Tetanusanatoxin, die in einer Impfdosis vorliegt, 10 bis 25 µg beträgt.

3. Verwendung gemäß Anspruch 1 oder 2 einer Zusammensetzung, worin "n" zwischen 4 und 11 liegt.

4. Verwendung gemäß Anspruch 3 einer Zusammensetzung, worin "n" gleich 11 ist und worin die "n" Kapselpolysaccharide der "n" Polysaccharid-Konjugate die Polysaccharide der Serotypen 1, 3, 4, 5, 6B, 7F, 9V, 14, 18C, 19F und 23F sind.

5. Verwendung einer Zusammensetzung, die "p" Polysaccharid-Konjugate unfasst, die jeweils aus einem an das Diphtherieanatoxin gekoppelten Kapselpolysaccharid von *Streptococcus pneumoniae* bestehen, zur Herstellung eines Impfstoffs, der gegen die Infektionen mit *Corynebacterium diphtheriae* schützt, wobei in der Zusammensetzung:
*(1)* die "p" Kapselpolysaccharide der "p" Polysaccharid-Konjugate alle verschieden sind,
*(2)* "p" größer oder gleich 2 und kleiner oder gleich 15 ist,
*(3)* die Gesamtmenge an konjugiertem Diphtherieanatoxin, die in einer Impfdosis vorliegt, 40 bis 130 µg beträgt und
*(4)* die Gesamtmenge an konjugiertem Diphtherieanatoxin, die in einer Impfdosis vorliegt, ausreicht, um einen Schutz gegen die Infektionen mit *Corynebacterium diphtheriae* zu induzieren.

6. Verwendung gemäß Anspruch 5 einer Zusammensetzung, worin die Gesamtmenge an konjugiertem Diphtherieanatoxin, die in einer Impfdosis vorliegt, 40 bis 85 µg beträgt.

7. Verwendung gemäß Anspruch 5 oder 6 einer Zusammensetzung, worin "p" zwischen 4 und 15 liegt.

8. Verwendung gemäß Anspruch 7 einer Zusammensetzung, worin "p" gleich 11 ist und worin die "p" Kapselpolysaccharide der "p" Polysaccharid-Konjugate die Polysaccharide der Serotypen 1, 3, 4, 5, 6B, 7F, 9V, 14, 18C, 19F und 23F sind.

9. Verwendung einer Zusammensetzung, die "n" Polysaccharid-Konjugate, die jeweils aus einem an das Tetanusanatoxin gekoppelten Kapselpolysaccharid von *Streptococcus pneumoniae* bestehen, und "p" Polysaccharid-Konjugate, die jeweils aus einem an das Diphtherieanatoxin gekoppelten Kapselpolysaccharid von *Streptococcus pneumoniae* bestehen, umfasst, zur Herstellung eines Impfstoffs, der gegen die Infektionen mit *Clostridium tetani* und mit *Corynebacterium diphtheriae* schützt, wobei in der Zusammensetzung:
*(1)* die "n" Kapselpolysaccharide der Gruppe der "n" Polysaccharid-Konjugate alle voneinander verschieden sind und die "p" Kapselpolysaccharide der Gruppe der "p" Polysaccharid-Konjugate alle voneinander verschieden sind,
*(2)* "n" größer oder gleich 2 und kleiner oder gleich 23 ist,
*(3)* "p" größer oder gleich 2 und kleiner oder gleich 15 ist,
*(4)* die Gesamtmenge an konjugiertem Tetanusanatoxin, die in einer Impfdosis vorliegt, 6 bis 40 µg beträgt,
(*5*) die Gesamtmenge an konjugiertem Diphtherieanatoxin, die in einer Impfdosis vorliegt, 40 bis 130 µg beträgt und
*(6)* die Gesamtmengen an konjugiertem Tetanus- und Diphtherieanatoxin, die in einer Impfdosis vorliegen, ausreichen, um einen Schutz gegen die Infektionen mit *Clostridium tetani* und mit *Corynebacterium diphtheriae* zu induzieren.

10. Verwendung gemäß Anspruch 9 einer Zusammensetzung, worin die "n" Kapselpolysaccharide mit den "p" Kapselpolysacchariden identisch sind.

11. Verwendung gemäß Anspruch 9 einer Zusammensetzung, worin die "n" Kapselpolysaccharide von den "p" Kapselpolysacchariden verschieden oder teilweise verschieden sind.

12. Verwendung gemäß Anspruch 11 einer Zusammensetzung, worin:
*(1)* n zwischen 2 und 9 liegt,
*(2)* p zwischen 2 und 11-n liegt,
*(3)* die "n" Kapselpolysaccharide von den "p" Kapselpolysacchariden alle verschieden sind und ausgewählt sind aus der Gruppe der Kapselpolysaccharide der Serotypen 1, 3, 4, 5, 6B, 7F, 9V, 14, 18C, 19F und 23F.

13. Verwendung gemäß Anspruch 12 einer Zusammensetzung, worin:
*(1)* "n" gleich 7 ist,
*(2)* "p" gleich 4 ist,
*(3)* die "n" Kapselpolysaccharide die Kapselpolysaccharide der Serotypen 1, 4, 5, 7F, 9V, 19F und 23F sind und
*(4)* die "p" Kapselpolysaccharide die Kapselpolysaccharide der Serotypen 3, 6B, 14 und 18C sind.

## Claims

1. Use of a composition comprising "n" polysaccharide conjugates consisting individually of a *Streptococcus pneumoniae* capsular polysaccharide coupled to the tetanus toxoid, for manufacturing a vaccine which protects against *Clostridium tetani* infections, in which composition:
(*1*) the "n" capsular polysaccharides of the "n" polysaccharide conjugates are all different,
(*2*) "n" is greater than or equal to 2 and less than or equal to 23,
(*3*) the total amount of conjugated tetanus toxoid present in one vaccine dose is from 6 to 40 µg; and
(*4*) the total amount of conjugated tetanus toxoid present in one vaccine dose is sufficient to induce protection against *Clostridium tetani* infections.

2. Use according to Claim 1, of a composition in which the total amount of conjugated tetanus toxoid present in one vaccine dose is from 10 to 25 µg.

3. Use according to Claim 1 or 2, of a composition in which "n" is between 4 and 11.

4. Use according to Claim 3, of a composition in which "n" is equal to 11 and in which the "n" capsular polysaccharides of the "n" polysaccharide conjugates are the polysaccharides of the serotypes 1, 3, 4, 5, 6B, 7F, 9V, 14, 18C, 19F and 23F.

5. Use of a composition comprising "p" polysaccharide conjugates consisting individually of a *Streptococcus pneumoniae* capsular polysaccharide coupled to the diphtheria toxoid, for manufacturing a vaccine which protects against *Corynebacterium diphtheriae* infections, in which composition:
(*1*) the "p" capsular polysaccharides of the "p" polysaccharide conjugates are all different,
(*2*) "p" is greater than or equal to 2 and less than or equal to 15,
(*3*) the total amount of conjugated diphtheria toxoid present in one vaccine dose is from 40 to 130 µg; and
(*4*) the total amount of conjugated diphtheria toxoid present in one vaccine dose is sufficient to induce protection against *Corynebacterium diphtheriae* infections.

6. Use according to Claim 5 of a composition in which the total amount of conjugated diphtheria toxoid present in one vaccine dose is from 40 to 85 µg.

7. Use according to Claim 5 or 6, of a composition in which "p" is between 4 and 15.

8. Use according to Claim 7, of a composition in which "p" is equal to 11 and in which the "p" capsular polysaccharides of the "p" polysaccharide conjugates are the polysaccharides of the serotypes 1, 3, 4, 5, 6B, 7F, 9V, 14, 18C, 19F and 23F.

9. Use of a composition comprising "n" polysaccharide conjugates consisting individually of a *Streptococcus pneumoniae* capsular polysaccharide coupled to the tetanus toxoid and "p" polysaccharide conjugates consisting individually of a *Streptococcus pneumoniae* capsular polysaccharide coupled to the diphtheria toxoid, for manufacturing a vaccine which protects against *Clostridium tetani* infections and *Corynebacterium diphtheriae* infections, in which composition:
(*1*) the "n" capsular polysaccharides of the group of the "n" polysaccharide conjugates are all different from one another and the "p" capsular polysaccharides of the group of the "p" polysaccharide conjugates are all different from one another,
(*2*) "n" is greater than or equal to 2 and less than or equal to 23,
(*3*) "p" is greater than or equal to 2 and less than or equal to 15,
(*4*) the total amount of conjugated tetanus toxoid present in one vaccine dose is from 6 to 40 µg,
(*5*) the total amount of conjugated diphtheria toxoid present in one vaccine dose is from 40 to 130 µg; and
(*6*) the total amounts of conjugated tetanus toxoid and conjugated diphtheria toxoid present in one vaccine dose are sufficient to induce protection against *Clostridium tetani* infections and *Corynebacterium diphtheriae* infections.

10. Use according to Claim 9, of a composition in which the "n" capsular polysaccharides are identical to the "p" capsular polysaccharides.

11. Use according to Claim 9, of a composition in which the "n" capsular polysaccharides are different or partially different from the "p" capsular polysaccharides.

12. Use according to Claim 11, of a composition in which:
(*1*) n is between 2 and 9,
(*2*) p is between 2 and 11-n,
(*3*) the "n" capsular polysaccharides are all different from the "p" capsular polysaccharides and are chosen from the group of the capsular polysaccharides of the serotypes 1, 3, 4, 5, 6B, 7F, 9V, 14, 18C, 19F and 23F.

13. Use according to Claim 12, of a composition in which:
(*1*) "n" is equal to 7,
(*2*) "p" is equal to 4,
(*3*) the "n" capsular polysaccharides are the capsular polysaccharides of the serotypes 1, 4, 5, 7F, 9V, 19F and 23F, and
(*4*) the "p" capsular polysaccharides are the capsular polysaccharides of the serotypes 3, 6B, 14 and 18C.
